# EUROPEAN PATENT APPLICATION

(11) **EP 2 071 324 A1**
(43) Date of publication of application: **17.06.2009**
(21) Application number: 07807510.8
(22) Date of filing: 19.09.2007
(51) Int. Cl.: G01N 27/12

(54) **HYDROGEN SENSOR**

(30) Priority: 28.09.2006 JP 2006265729
(71) Applicant: MIKUNI CORPORATION, Chiyoda-ku, Tokyo 101-0021 (JP)
(72) Inventor: FUKUI, Katsuhiko, Iwate-gun, Iwate 0200188 (JP); HIRAYAMA, Toshinori, Iwate-gun, Iwate 0200188 (JP)
(74) Representative: Gulde Hengelhaupt Ziebig & Schneider
(86) International application number: PCT/JP2007/068123
(87) International publication number: WO 2008/038547

(57) **Abstract**

A hydrogen sensor comprising:
a substrate,
a detection film which is formed on the substrate and is composed of a rare earth metal,
a protection film which is formed on the detection film and is a dispersion of hydrogen-permeable metal particles in a ceramic material, and
a pair of electrodes which is formed apart on the protection film and are electrically connected with the detection film via the protection film. The hydrogen-permeable metal particles are Pd, Pt, Nb, V and Ta and the preferable content thereof is 20 to 70% by mass. It is preferred that the resistance of the protection film between the electrodes is 10 to 100,000 Ω and that the resistance of the detection film in the interelectrode direction is smaller than the resistance of the protection film in the interelectrode direction.

## Description

### [Technical Field]

The present invention relates to a hydrogen sensor for detecting hydrogen gases of relatively low concentration to high concentration. As the hydrogen gas of relatively low concentration, there are mentioned, for example, hydrogen gases leaking from hydrogen gas-treating apparatuses such as fuel cell for automobile, fuel cell for home use, and the like. As the hydrogen gas of relatively high concentration, there are mentioned, for example, high-concentration hydrogen gases in hydrogen gas-treating apparatuses. The present invention relates to a hydrogen sensor suitably used for the detection, control, etc. of hydrogen gases of various concentration levels.

### [Background Art]

As gas sensor, there are widely known those having a structure comprising a detection film and element electrodes for detecting the change of the output of the detection film. The output of the detection film is influenced, in many cases, by gas components other than the gas component to be detected. In order to eliminate this influence, there have been used gas sensors in which the surface of the detection film is covered with a protection film allowing for selective permeation of a to-be-detected gas therethrough.

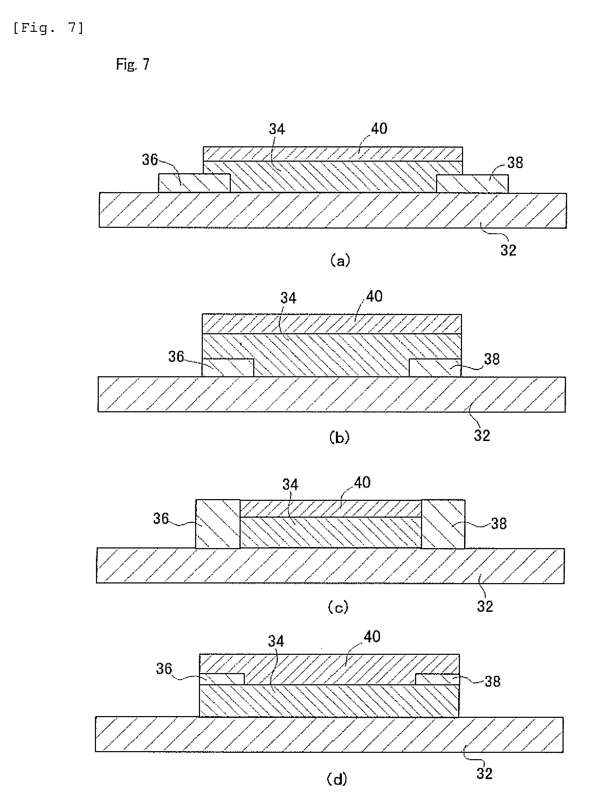

The structures of conventional gas sensors in which the surface of the detection film is covered with a protection film, are shown in Figs. 7(a) to 7(d). All of these conventional gas sensors comprise a substrate 32 of electrical insulation, a detection film 34 formed on the surface of the substrate 32 in close adhesion, and element electrodes 36 and 38 formed at the both ends of the detection film 34. 40 is a protection film or a catalyst layer, formed on the surface of the detection film 34.

In Patent Literature 1 is described a gas sensor comprising a substrate, a pair of electrodes, a detection film, a catalyst layer for enhancing the detection sensitivity of a to-be-detected gas, and a protection film capable of selectively burning gas components other than the to-be-detected gas, with the electrodes to the protection film being formed on the substrate in the above order. This gas sensor has a basic structure of the gas sensor shown in Fig. 7(a) although it is different from the latter gas sensor in that it has a catalyst layer.

With respect to the sensor for detection of hydrogen gas, various proposals have been made. For example, there is a proposal on a hydrogen sensor using, as a hydrogen detection film, a thin film of rare earth metal such as yttrium (Y), lanthanum (La) or the like (Patent Literature 2).

In this proposal, the change of the physical property of rare earth metal, which appears when the rare earth metal is exposed to hydrogen, is utilized for detection of hydrogen. The rare earth metal (M) constituting the detection film has a catalytic action of adsorbing hydrogen gas and generating proton (H⁺) and electron (e⁻). At this time, the rare earth metal per se reacts with the proton generated and becomes MHx. Consequently, the resistance of the detection film changes.

The rare earth metal is adversely influenced by components other than hydrogen, present together with the hydrogen, such as nitrogen, oxygen, ammonia, hydrocarbons and the like. In the sensor, in order to prevent the influence, the surface of the rare earth metal film is covered with a protection film composed of palladium (Pa), platinum (Pt), an alloy thereof, or the like, all having hydrogen permeability.

A hydrogen-permeable metal such as Pd, Pt or the like repeats volume expansion and contraction when, in hydrogen permeation therethrough, it absorbs and releases hydrogen. This expansion and contraction causes the mechanical stress of the hydrogen-permeable metal, whereby the hydrogen-permeable metal is deteriorated and gives rise to cracking, etc. For this reason, the hydrogen sensor using a hydrogen-permeable metal in the protection film is inferior in durability.

The hydrogen-permeable metal such as Pd, Pt or the like tends to diffuse into the rare earth metal constituting the detection film, such as yttrium (Y), lanthanum (La) or the like. The progress of this diffusion leads to a reduction in the hydrogen detectability of rare earth metal with the passage of time. For this reason, the hydrogen sensor using a hydrogen-permeable metal in the protection film has a problem in durability.

Further, since Pd or Pt is a very expensive metal, the hydrogen sensor using such a metal in the protection film incurs a high production cost.

The present inventors made a study in order to solve the above problems. As result, the present inventors thought of an idea of using, as a protection film, a ceramic material having hydrogen-permeable metal particles dispersed therein and filed a patent on a hydrogen sensor using such a protection film (Patent Literature 3). The hydrogen-permeable metal particles used in the hydrogen sensor are palladium (Pa), platinum (Pt), niobium (Nb), vanadium (V) or tantalum (Ta), and the ceramic material is AlNx₁, AlOx₂, SiNx₃ and SiOx₄ in which 0.5≦x₁≦₁, 0.8≦x₂≦1.5, 0.7≦x₃≦1.3 and 1≦x₄≦2. As the electrode material, there are used gold, platinum, palladium, titanium, aluminum, copper, silver, etc.

This hydrogen sensor, as compared with conventional sensors using, as the protection film, a thin film of Pd or Pt, is low in the diffusion of the hydrogen-permeable metal particles dispersed in the protection film, into the detection film. Consequently, the hydrogen sensor is superior in durability and gives a low production cost.

However, when a sensor having a general element structure shown in any of Figs. 7(a) to 7(d) is produced using the above-mentioned protection film having hydrogen-permeable metal particles dispersed therein, there are the following problems. That is, in any of the conventional elements shown in Figs. 7(a) to 7(d), the detection film and the electrodes are in direct contact with each other. Therefore, there appears, between the detection film and the electrodes, mutual diffusion between the rare earth metal and the electrode-constituting metal.

In the sensor structures described in Figs. 7(a) and 7(b), a detection film is laminated on a substrate on which a pair of thin-film electrodes is formed. The thickness of each electrode is required to be 10 nm or more. Resultantly, the detection film has an offset structure having notches of a depth corresponding to the electrodes. Similarly, the sensor of Fig. 7(d) shows a structure having notches at the two ends of the protection film for engagement of the electrodes formed on the detection film. Fig. 7(c) shows a structure in which the positions of each two ends of the detection film and the protection film are fixed by the electrodes formed so as to contact with the each two ends. These structures undergo thermal stress easily when the detection film and the protection film expand or contract owing to factors such as heat and the like. As a result, the detection film and the protection film tend to cause cracking.

Incidentally, as other conventional technique relevant to the present invention, there is a hydrogen sensor provided with a detection film composed of metal oxide particles loaded, on the surfaces, with fine particles of Pt, Pd or the like (Patent Literature 4). In this sensor, the protection film showing high selectivity to hydrogen gas is not formed on the detection film. The hydrogen sensor of the present invention described later is different from the hydrogen sensor described in the Patent Literature 4 because, in the present hydrogen sensor, the detection film is constituted by a thin film of rare earth metal and the protection film showing high selectivity to hydrogen gas is formed on this detection film.
Patent Literature 1: Japanese Patent Application laid open No. 1999-183420 (Claims, Fig. 2)
Patent Literature 2: National Publication of International Patent Application No. 2002-535651 (Claims)
Patent Literature 3: Japanese Patent Application laid open No. 2005-274559 (Claims)
Patent Literature 4: Japanese Patent Application laid open No. 2006-162365 (Claims)

### [Disclosure of the Invention]

### [Task to Be Achieved by the Invention]

The present invention has been made in view of the above situation. The present invention aims at providing a hydrogen sensor comprising, as the protection film, a ceramic material having hydrogen-permeable metal particles dispersed therein, which hydrogen sensor hardly causes cracking by thermal stress, etc., hardly causes mutual diffusion of different metals between the protection film and the detection film and, as a result, has high durability.

### [Means for Achieving the Task]

The present inventors made a study in order to achieve the above task. As a result, it was found that, by forming electrodes on a protection film without contacting with a detection film, the electrodes and the detection film could have electrical connection via the protection film. Further, it was confirmed that the present sensor could detect hydrogen gas accurately. These have led to the completion of the present invention.

The present invention is described below.
[1] A hydrogen sensor comprising:
   a substrate,
   a detection film which is formed on the substrate and is composed of a rare earth metal,
   a protection film which is formed on the detection film and is a dispersion of hydrogen-permeable metal particles in a ceramic material, and
   a pair of electrodes which is formed apart on the protection film and are electrically connected with the detection film via the protection film.
[2] The hydrogen sensor according to [1], wherein the resistance of the protection film between the electrodes is 10 to 100,000 Ω.
[3] The hydrogen sensor according to [1], wherein the resistance of the detection film between the electrodes is smaller than the resistance of the protection film between the electrodes.
[4] The hydrogen sensor according to [1], wherein the resistance of the detection film between the electrodes is 0.1 to 1,000 Ω.
[5] The hydrogen sensor according to [1], wherein the thickness of the detection film is 5 to 1,000 nm and the thickness of the protection film is 5 to 100 nm.
[6] The hydrogen sensor according to [1], wherein the content of the hydrogen-permeable metal particles in the protection film is 20 to 70% by mass.
[7] The hydrogen sensor according to [1], wherein the detection film is composed of at least one member selected from the group consisting of yttrium, cerium and lanthanum.
[8] The hydrogen sensor according to [1], wherein the hydrogen-permeable metal particles are composed of at least one member selected from the group consisting of palladium (Pd), platinum (Pt), niobium (Nb), vanadium (V) and tantalum (Ta).
[9] The hydrogen sensor according to [1], wherein the ceramic material is composed of at least one member selected from the group consisting of AlNx₁, AlOx₂, SiNx₃ and SiOx₄ in which 0.5≦x₁≦1, 0.8≦x₂≦1.5, 0.7≦x₃≦1.3 and 1≦x₄≦2.
[10] The hydrogen sensor according to [1], wherein the substrate is made of a glass plate, a ceramic plate or a single crystal plate.
[11] The hydrogen sensor according to [1], wherein the electrodes are formed with at least one member selected from the group consisting of gold, platinum, palladium, titanium, aluminum, copper and silver.

### [Effect of the Invention]

In the hydrogen sensor of the resent invention, the electrodes and the detection film are in electrical connection via the protection film. Therefore, there is no mutual diffusion of different metals between the electrodes and the detection film. The protection film is a near uniform dispersion of hydrogen-permeable metal particles in a ceramic material; therefore, mutual dispersion of different metals are low between the protection film and the detection film. Consequently, there is no reduction in the sensitivity of hydrogen sensor over a long period.

In the hydrogen sensor of the present invention, a detection film, a protection film and electrodes are formed on a substrate in this order, and the present hydrogen sensor has a uniform structure in the substrate surface direction. Therefore, the detection film and the protection film hardly undergo thermal stress and are resistant to cracking, etc.

In the protection film, the mechanical stress caused by the volume change of the hydrogen-permeable metal particles which appears in the absorption and release of hydrogen by the hydrogen-permeable metal particles, is received by the ceramic material of high rigidity and absorbed thereby. Consequently, the deterioration of the protection film, caused by the bonding of the hydrogen-permeable metal with hydrogen is low.

Accordingly, the present hydrogen sensor having the above-mentioned structure has high selectivity to hydrogen gas, is low in deterioration of detection film by non-hydrogen gases, and can withstand long-term use.

### [Brief Description of the Drawings]

Fig. 1 is a sectional view showing an example of the hydrogen sensor of the present invention.
Fig. 2 is a plan view of the hydrogen sensor shown in Fig. 1.
Fig. 3 is a graph showing a relation between the thickness and resistance of detection film.
Fig. 4 is a graph showing relations between the content of hydrogen-permeable metal particles in protection film and the resistance of protection film.
Fig. 5 is a graph showing the change of resistance to hydrogen gas concentration, of the hydrogen sensor obtained in Example 1.
Fig. 6 is a graph showing the changes of basic resistances to exposure times, of the hydrogen sensors obtained in Example 2 and Comparative Example 1.
Figs. 7(a) to 7(d) are sectional views showing the structures of conventional hydrogen sensors.

### [Explanation of Symbols]

1 is a hydrogen sensor; 2 is a substrate; 3 is a detection film; 5 is a protection film; 7 and 9 are each an electrode; 13 is a hydrogen-permeable metal particle; 15 is a ceramic matrix; d is a distance between electrodes; t₃ is the thickness of detection film; and t₅ is the thickness of protection film.

### [Best Mode for Carrying out the Invention]

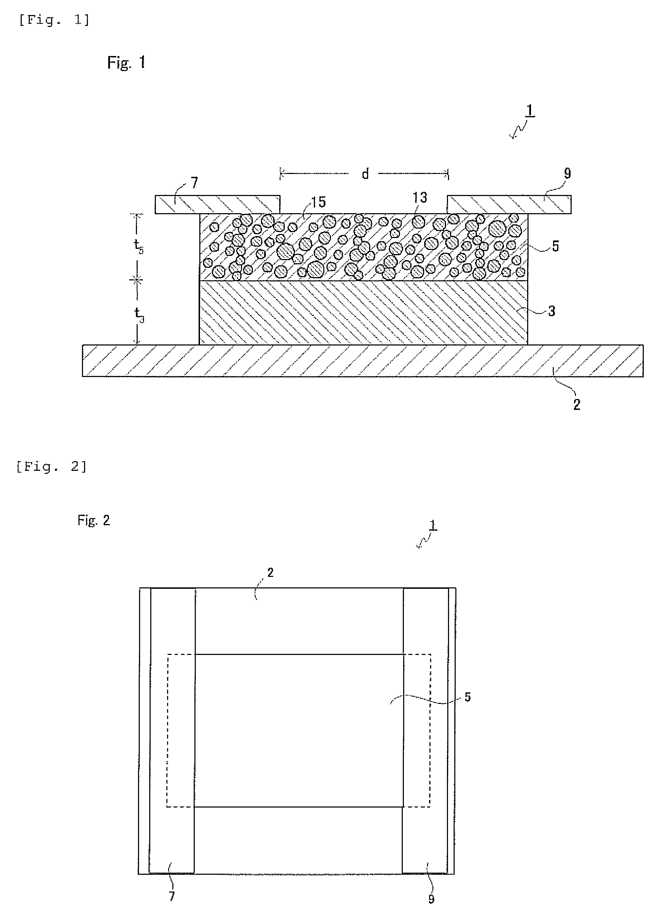

Fig. 1 is a sectional view showing an example of the hydrogen sensor of the present invention, and Fig. 2 is a plan view of the hydrogen sensor shown in Fig. 1. In Fig. 1, 1 is a hydrogen sensor and 2 is a substrate. On one side (the upper side in Fig. 1) of the substrate 2 is formed a hydrogen detection film 3 composed of a rare earth metal. On the upper side of the detection film 3 is formed a protection film 5. The protection film 5 is a near uniform dispersion of a large number of hydrogen-permeable metal particles 13 in a matrix constituted by a ceramic material 15.

The particle diameters of the hydrogen-permeable metal particles 13 are 1 to 10 nm, preferably 2 to 6 nm. The particle diameters are smaller than the thickness t₅ of the protection film 5. In Fig. 1, t₃ is the thickness of the detection film 3.

At the two ends of the upper side of the protection film 5 is formed a pair of rectangular electrodes 7 and 9 in parallel and face to face. The electrodes 7 and 9 are in contact only with the protection film 5 and partially stretch horizontally from the upper side of the protection film 5. However, the protection film 5 is in no direct contact with the detection film 3.

The electrical connection between the electrodes 7 and 9 and the detection film 3 is achieved via the conductive paths (not shown) formed by the mutual contact of the large number of hydrogen-permeable metal particles 13 dispersed in the protection film 5.

As described later, the thickness t₅ of the protection film is preferably 5 to 100 nm; therefore, in production of the present sensor, it is practically impossible that the distance d between the electrodes, shown in Fig. 1 is smaller than the thickness of the protection film.

The resistance of the detection film 3 between the electrodes (hereinafter, the resistance is abbreviated as the resistance of the detection film, in some cases) is preferably smaller than the resistance of the protection film 5 between the electrodes (hereinafter, the resistance is abbreviated as the resistance of the protection film, in some cases).

Here, the resistance of the detection film is a value measured by the following method.

First, a detection film is formed on an insulating substrate. Then, thereon are formed a protection film [thickness: 10 nm, Pd content: 20%, matrix: SiNx₃ (x₃ = 1.0)] and a pair of electrodes. The resistance of the formed protection film between the two electrodes is measured at 25°C in a hydrogen-free atmosphere. The resistance obtained by the measurement is taken as the resistance of the detection film between the electrodes.

The resistance of the protection film between the electrodes is measured by the following method. First, a protection film 5 alone is formed on a substrate. Thereon is formed a pair of electrodes similar to the above-mentioned electrodes 7 and 9. Then, the resistance between the electrodes is measured. The measurement is conducted at 25°C in a hydrogen-free atmosphere. The value obtained is taken as the resistance of the protection film.

Upon contact of hydrogen gas with the detection film 3, the detection film 3 increases its resistance. When the resistance of the detection film 3 is same as or larger than the resistance of the protection film 5 and when hydrogen gas is in no contact with the detection film, an electric current tends to flow through the protection film in preference to through the detection film. Then, when hydrogen gas comes into contact with the detection film, the detection film increases its resistance. As a result, the electric current flowing through the detection film 3 decreases. When the electric current flowing through the detection film 3 is small, the composite resistance of the protection film 5 and the detection film 3 is close to the resistance of the protection film 5. Therefore, in this case, it is difficult to detect the change of the resistance of the detection film.

Meanwhile, when the protection film 5 has a sufficiently large resistance as compared with the detection film 3, an electric current tends to flow preferentially through the detection film. In this case, the composite resistance of the protection film 5 and the detection film 3 is close to the resistance of the detection film. Then, upon contact of hydrogen with the detection film, the resistance of the detection film increases. This change of the resistance of the detection film can be detected until the resistance of the detection film becomes close to the resistance of the protection film. When the resistance of the detection film becomes equal to or larger than the resistance of the protection film, the composite resistance of the two films becomes close to the resistance of the protection film.

Accordingly, the resistance of the detection film 3 between the electrodes is preferred to be sufficiently smaller than the resistance of the protection film 5 between the electrodes. Specifically, the resistance of the detection film 3 between the electrodes is preferably 1.25 to 50%, more preferably 2.5 to 15% of the resistance of the protection film 5 between the electrodes.

The resistance of the protection film 5 between the electrodes is preferably 10 to 100,000 Ω, more preferably 10 to 10,000 Q, particularly preferably 100 to 5,000 Ω. In the protection film 5, a large number of hydrogen-permeable metal particles 13 are dispersed therein in near uniformity. As a result, by controlling the thickness of the protection film 5 in the above-mentioned range and the resistance of the protection film 5 between the electrodes at 10 Ω or more, the electric current flowing through the detection film can be made sufficiently large. Consequently, the change of the resistance of the detection film can be detected reliably.

When the resistance of the protection film between the electrodes is less than 10 Ω, the resistance of the detection film per se needs to be set even smaller. However, this invites the difficulty in production. Moreover, the change of the resistance of the detection film when the film comes into contact with hydrogen, is small. When the resistance of the protection film between the electrodes is larger than 100,000 Ω, it is difficult to detect the change of the resistance of the detection film via the protection film.

The resistance of the detection film 3 (formed with a rare earth metal) between the electrodes is preferably 0.1 to 1,000 Ω, more preferably 1 to 800 Q, particularly preferably 10 to 500 Ω. A detection film having a resistance of less than 0.1 Ω between electrodes is difficult even to produce. With a detection film having a resistance of more than 1,000 Ω in an interelectrode direction, the electric current flowing therethrough is small. As a result, such a hydrogen sensor is inferior in detectability of hydrogen gas.

On other side (the lower side in Fig. 1) of the substrate 2 may be formed a heater (not shown) made with a metal resistor. The metal resistor is preferably a thin film resistor. As the thin film resistor, there is preferred a thin film resistor which is composed of platinum, ruthenium oxide, silver-palladium alloy or the like and which is formed on the other side of the substrate in an intended pattern.

### (Substrate)

As the substrate, there can be used a smooth, insulating and gas-impermeable substrate such as glass plate, ceramic plate or single crystal plate of sapphire, zinc oxide (ZnO), magnesium oxide (MgO) or the like.

### (Detection film)

The detection film 3 is composed mainly of a rare earth metal having hydrogen detectability. As the rare earth metal, there is preferred at least one member selected from the group consisting of yttrium, cerium and lanthanum because they are superior in hydrogen detectability. As the method for producing the detection film, there are, for example, a method of allowing a rare earth metal to giving rise to gas phase growth on a substrate in a vacuum atmosphere and a method of giving rise to sputtering in an argon atmosphere to form a film.

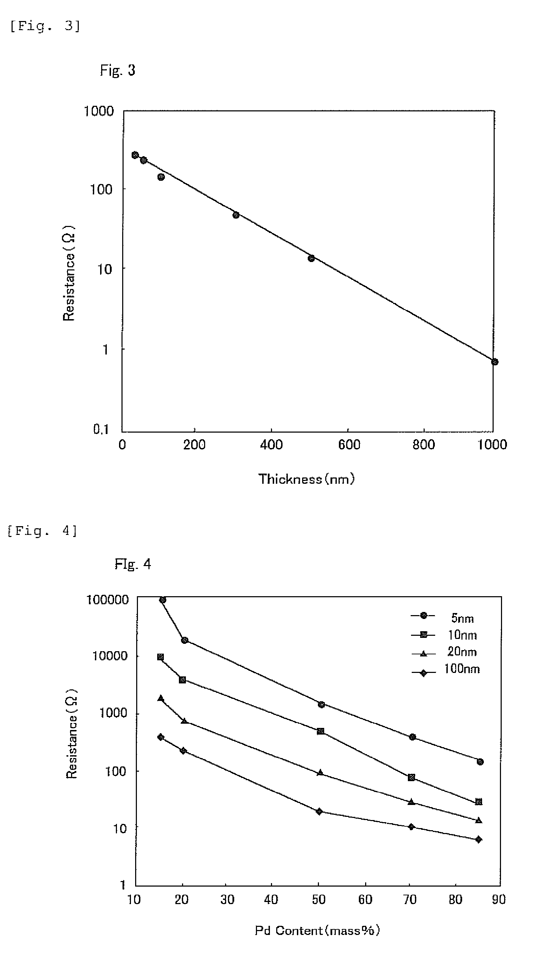

Fig. 3 shows an example of the relation between the thickness and resistance, of a detection film. This detection film is a film formed on a ceramic substrate using yttrium. In this detection film composed of yttrium, the resistance tends to decrease as the film thickness increases.
A detection film of less than 0.1 Ω is difficult even to produce. In a detection film of more than 1,000 Ω, the electric current flowing therethrough is small and the hydrogen gas detectability is low.

Therefore, the film thickness of the detection film is preferably 5 to 1,000 nm. By controlling the thickness of the detection film in this range, the resistance of the detection film can be kept in a range of 0.1 to 1,000 Ω. Incidentally, when the film thickness is less than 5 nm, the detection film may be insufficient in strength. Meanwhile, when the thickness is more than 1,000 nm, the resistance of the detection film does not show a decrease in parallel to the increase in film thickness. Meanwhile, the increase in film thickness generates various problems such as reduction in response, associated with increase in detection film capacity, reduction in sensitivity, increase in production cost, and the like.

The thickness of the detection film is preferably determined depending upon the purpose of detection. When the to-be-measured concentration of hydrogen is relatively low, for example, 1% by volume or less, in particular, less than 5,000 ppm, the thickness of the detection film is preferably less than 100 nm, more preferably less than 50 nm, particularly preferably 5 to 40 nm. As the usage of a hydrogen sensor for detecting hydrogen gas of low concentration of less than 5,000 ppm, there can be mentioned, for example, a hydrogen gas leakage detector.

When the to-be-measured concentration of hydrogen is high, for example, higher than 1% by volume, in particular, 5% by volume or more, the thickness of the detection film is preferably 100 nm or more, more preferably 300 to 500 nm. As the usage of a hydrogen sensor for detecting hydrogen gas of high concentration, there can be mentioned, for example, a hydrogen sensor used for control of the hydrogen gas concentration inside an apparatus using hydrogen.

### (Protection film)

The protection film 5 shown in Fig. 1 is a thin film of a dispersion of a large number of hydrogen-permeable metal particles 13 in a ceramic material 15.

Relations between palladium content and resistance, of protection films which are each a dispersion of palladium particles used as hydrogen-permeable metal particles 13, are shown in Fig. 4. Fig. 4 shows relations between palladium content and resistance, of 4 kinds of protection films (samples) of different film thicknesses each formed on a ceramic substrate. Fig. 4 indicates a tendency that the resistance of each protection film decreases as the content of palladium increases. Fig. 4 also indicates a tendency that the resistance of protection film decreases as the film thickness of protection film increases.

The content of the hydrogen-permeable metal particles in the protection film is preferably 20 to 70% by mass, more preferably 40 to 60% by mass. By controlling the content of the hydrogen-permeable metal particles in this range, the resistance of the protection film can be kept at 10 to 100,000 Ω. When the content of the hydrogen-permeable metal particles is less than 20% by mass, the amount of hydrogen gas permeable through the protection film decreases and the ability of the protection film, of feeding hydrogen gas into the detection film is insufficient. Further, the electrical connection between the electrodes 7 and 9 and the detection film 3 is insufficient. Meanwhile, when the content of the hydrogen-permeable metal particles is more than 70% by mass, the deterioration of the protection film caused by its volume change appearing when the hydrogen-permeable metal particles bond with hydrogen, is striking. Further, the diffusion of the hydrogen-permeable metal atoms into the detection film is striking. As a result, the hydrogen detectability of the detection film is striking low.

Furthermore, in the protection film in which the content of the hydrogen-permeable metal particles is more than 70% by mass, its mechanical strength is insufficient when its film thickness is small. Therefore, it is difficult to produce a protection film having a film thickness of 5 to 100 nm.

As the hydrogen-permeable metal particles constituting the protection film, there is preferred at least one kind of metal particles selected from the group consisting of palladium (Pd), platinum (Pt), niobium (Nb), vanadium (V) and tantalum (Ta). The hydrogen-permeable metal particles may be an alloy of such a metal. Of them, an alloy of Pd is preferred particularly.

These hydrogen-permeable metal particles 13 may be dispersed in a ceramic material as particles of simple metal element, or may be dispersed as particles of the above-mentioned alloy. As the alloy of hydrogen-permeable metal, there can be used an alloy which has been used in hydrogen-permeable film. As the alloy element, there can be mentioned, for example, calcium, iron, copper, vanadium, nickel, titanium, chromium and zirconium.

The protection film can be formed, for example, by a method of allowing a ceramic material and a hydrogen-permeable metal to giving rise to simultaneous gas-phase growth on the detection film, or by a simultaneous sputtering method. When the ceramic material is AlNx₁, a protection film can be formed by using Al metal (as a film material) and a hydrogen-permeable metal and conducting film formation in a nitrogen gas atmosphere. When the ceramic material is AlOx₂ a protection film can be formed by using Al metal (as a film material) and a hydrogen-permeable metal and conducting film formation in an oxygen gas atmosphere. When the ceramic material is SiNx₃ or SiOx₄, a protection film can be formed by conducting film formation in the same manner as in the case of AlNx₁ or AlOx₂ except that silicon is used in place of the Al metal.

The protection film can be formed, for example, by using a composite target comprising an aluminum target and a Pd chip mounted on the aluminum target, having a smaller area than the aluminum target, fixing a substrate above the target, and, in this state, sputtering the composite target. The sputtering is conducted in a mixed gas atmosphere of argon and nitrogen. By forming, in this manner, a film on a substrate on which a detection film has been formed, a protection film can be formed on the detection film. The protection film formed is a mixture (a dispersion) of Pd (a hydrogen-permeable metal) and AlNx₁.

A protection film can also be formed by conducting two-way simultaneous sputtering in the same mixed gas atmosphere as in the above case, using an aluminum target and a Pd target.

The thickness of the protection film is preferably 5 to 100 nm, more preferably 7 to 40 nm. When the thickness of the protection film is less than 5 nm, the adverse effect of non-hydrogen components harmful to the detection film, such as nitrogen, oxygen, ammonia, hydrocarbons and the like may not be suppressed sufficiently. Meanwhile, when the thickness is more than 100 nm, the hydrogen permeability is insufficient. As a result, the accuracy of measurement of hydrogen gas concentration may be inferior. Or, the response time of hydrogen sensor may become long. Further, the reduction in production cost is small.

Incidentally, when Pd is used as the hydrogen-permeable metal, the thickness of the protection film can be made very small because Pd is superior in separability between hydrogen gas and other gases.

### (Ceramic material)

As the ceramic material 15 of Fig. 1 constituting a matrix layer in which hydrogen-permeable metal particles are dispersed, there can be used a nitride and/or an oxide, of aluminum (Al) or silicon (Si). Or, a silicide of a rare earth metal can be used.

Of these ceramic materials, preferred are AlNx₁, AlOx₂, SiNx₃ and SiOx₄ in which 0.5≦x₁≦1, 0.8≦x₂≦1.5, 0.7≦x₃≦1.3 and 1≦x₄≦2.

As the silicide of a rare earth metal, there can be mentioned silicides of yttrium (Y), lanthanum (La), etc.

Of these ceramic materials, AlNx₁ is preferred particularly. AlNx₁ has a high hardness and a high strength and therefore can effectively suppress the powdering of hydrogen-permeable metal particles, caused by their bonding with hydrogen.

### (Electrodes)

As the material for electrodes, there can be used conductive metals such as gold, platinum, palladium, titanium, aluminum, copper, silver and the like. Preferred electrode materials are gold, copper, platinum, etc.

As the method for formation of electrodes, a gas-phase growth method or a sputtering method can be used.

The thickness of each electrode is preferably 5 to 1,000 nm, more preferably 50 to 300 nm. When the thickness of the electrode is less than 5 nm, the formation per se of electrode is difficult and, when the thickness is more than 1,000 nm, the cost of production tends to be high. The shape of electrode, the electrode-to-electrode distance, etc. are set appropriately based on the shapes, sizes, etc. of protection film and detection film.

### (Method for production of hydrogen sensor)

The hydrogen sensor of the present invention can be produced, for example, by the following method. First, a detection film is formed on at least one side of a substrate by a gas-phase growth method or a sputtering method. Then, on the detection film is formed a protection film by a gas-phase growth method or a sputtering method. Thereafter, on the protection film is formed electrodes by a gas-phase growth method or a sputtering method.

Incidentally, the detection film is sensitive to non-hydrogen gases as well. Therefore, in order to accurately measure the concentration of hydrogen gas, it is preferred to completely segregate the detection film from the outside. In order to achieve it, it is preferred to conduct, in producing an actual product, a treatment such as complete coverage of detection film (not only the upper surface but also the side) with protection film, sealing detection film side with resin material of appropriate kind, and accommodation of hydrogen sensor in casing which can segregate the hydrogen sensor excluding protection film from the outside.

### [Industrial Applicability]

The hydrogen sensor of the present invention can be utilized in a sensor for detection of hydrogen gas leakage, used in a fuel cell automobile, a household fuel cell, etc.; a sensor for control of hydrogen gas concentration; a portable or stationary alarm device for hydrogen gas; a hydrogen gas densitometer; and so forth.

### [Examples]

### Example 1

Film formation was made on an alumina-made ceramic substrate of 25.4 mm in depth, 25.4 mm in width and 0.38 mm in thickness, using a special mask, using a high-frequency magnetron sputtering apparatus, to produce a hydrogen sensor shown in Fig. 1.

First, in a high-frequency magnetron sputtering apparatus were placed a ceramic substrate, an yttrium target and an aluminum target on which a palladium chip was mounted. The apparatus inside was made vacuum to 4x10⁻⁵ Pa. Then, argon gas was introduced into the apparatus (9.3x10⁻¹ Pa) and sputtering was conducted using the yttrium target at room temperature for 1 minute. As a result, an Y thin film (a detection film) of 1.3 mm in depth, 1.9 mm in width and 30 nm in thickness was formed on the ceramic substrate.

Next, into the apparatus were introduced argon gas and nitrogen gas (volume ratio = 85 : 15 Pa), and sputtering was conducted using the aluminum target having a palladium chip mounted thereon, at a pressure of 9.3x10⁻¹ Pa at room temperature for 1.5 minutes. As a result, a protection film of 10 nm in thickness was formed on the detection film. The formed protection film was a uniform dispersion of Pd particles (diameters: 2 to 6 nm) in an AlNx₁ (x₁ = 0.9) matrix. The Pd content in the protection film was 40 % by mass.

Thereafter, on the protection film was placed a mask having a pattern of element electrodes, and argon gas was introduced into the apparatus. Sputtering was conducted using a palladium chip at a pressure of 9.3x10⁻¹ Pa at room temperature for 3 minutes, to form a pair of element electrodes on the protection film. The formed electrodes were each 2.1 mm in depth, 0.42 mm in width and 200 nm in thickness. The distance between the electrodes was 1.5 mm.

In the hydrogen sensor obtained, the resistance of the Y thin film was 320 Ω and the resistance of the protection film was 1,000 Ω.

The hydrogen sensor obtained was assembled into a sensor casing and its portion excluding the protection film was segregated from the outside.

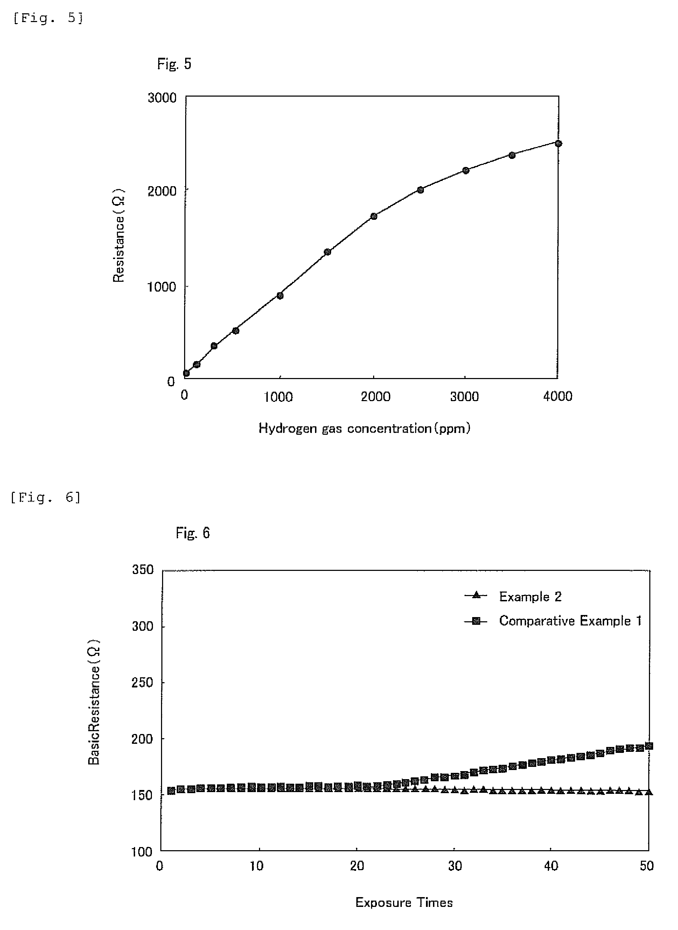

Using this hydrogen sensor, the hydrogen gas concentrations in mixed gases (mixtures with nitrogen gas) having hydrogen concentrations of 0 to 4,000 ppm were measured. The temperature of the hydrogen sensor was kept at 70°C. As a result, the hydrogen sensor could well detect hydrogen gases of low concentrations of less than 1% (detection of such a low concentration is required in detection of hydrogen gas leakage). In Fig. 5 is shown a graph indicating a relation between the hydrogen gas concentration of mixed gas and sensor resistance (i.e. composite resistance of protection film and detection film). Example 2

A hydrogen sensor was obtained in the same manner as in Example 1 except that the thickness of the Y thin film was 100 nm and a protection film [the matrix was SiNx₃ (x₃ = 1.0)] of 20 nm in thickness was formed on the Y thin film.
The resistance of the Y thin film was 160 Ω, and the resistance of the protection film was 300 Q. The concentrations of hydrogen gases were measured in the same procedure as in Example 1; as a result, the hydrogen sensor could well detect hydrogen gases of low concentrations of less than 1%.

### Comparative Example 1

There was obtained a hydrogen sensor having the same constitution as that of Example 2 except that the electrodes were formed between the substrate and the detection film, as shown in Fig. 7(b).

The hydrogen sensor of Example 2 and the hydrogen sensor of Comparative Example 1 were kept at 150°C and exposed to a hydrogen gas 100% atmosphere and the air 50 times. One time exposure cycle was 10 minutes in the air and 10 minutes in the hydrogen gas. After exposure to hydrogen gas, the resistance of the hydrogen sensor was measured in dry air (this was taken as basic resistance of sensor); and the change of this basic resistance was examined (Fig. 6). As a result, the basic resistance of the hydrogen sensor of Example 2 was almost constant (remained almost unchanged) even after 50 times of the exposure. Meanwhile, the basic resistance of the hydrogen sensor of Comparative Example 1 showed a change after 20 times of the exposure and increased gradually with an increase in the times of the exposure.

## Claims

1. A hydrogen sensor comprising:
a substrate,
a detection film which is formed on the substrate and is composed of a rare earth metal,
a protection film which is formed on the detection film and is a dispersion of hydrogen-permeable metal particles in a ceramic material, and
a pair of electrodes which is formed apart on the protection film and are electrically connected with the detection film via the protection film.

2. The hydrogen sensor according to Claim 1, wherein the resistance of the protection film between the electrodes is 10 to 100,000 Ω.

3. The hydrogen sensor according to Claim 1, wherein the resistance of the detection film between the electrodes is smaller than the resistance of the protection film between the electrodes.

4. The hydrogen sensor according to Claim 1, wherein the resistance of the detection film between the electrodes is 0.1 to 1,000 Ω.

5. The hydrogen sensor according to Claim 1, wherein the thickness of the detection film is 5 to 1,000 nm and the thickness of the protection film is 5 to 100 nm.

6. The hydrogen sensor according to Claim 1, wherein the content of the hydrogen-permeable metal particles in the protection film is 20 to 70% by mass.

7. The hydrogen sensor according to Claim 1, wherein the detection film is composed of at least one member selected from the group consisting of yttrium, cerium and lanthanum.

8. The hydrogen sensor according to Claim 1, wherein the hydrogen-permeable metal particles are composed of at least one member selected from the group consisting of palladium (Pd), platinum (Pt), niobium (Nb), vanadium (V) and tantalum (Ta).

9. The hydrogen sensor according to Claim 1, wherein the ceramic material is composed of at least one member selected from the group consisting of AlNx₁, AlOx₂, SiNx₃ and SiOx₄ in which 0.5≦x₁≦1, 0.8≦x₂≦1.5, 0.7≦x₃≦1.3 and 1≦x₄≦2.

10. The hydrogen sensor according to Claim 1, wherein the substrate is made of a glass plate, a ceramic plate or a single crystal plate.

11. The hydrogen sensor according to Claim 1, wherein the electrodes are formed with at least one member selected from the group consisting of gold, platinum, palladium, titanium, aluminum, copper and silver.
